# EUROPÄISCHE PATENTANMELDUNG

(11) **EP 2 420 560 A1**
(43) Veröffentlichungstag der Anmeldung: **22.02.2012**
(21) Anmeldenummer: 11003475.8
(22) Anmeldetag: 28.04.2011
(51) Int. Cl.: C12M 1/107, C12M 3/08

(54) **Anlage zur kontinuierlichen Verarbeitung von einen signifikanten Anteil an Phytomasse enthaltenden Materialien**

(30) Priorität: 18.08.2010 CZ 201023120 U
(71) Anmelder: Biomass Technology a.s., 76824 Hulin (CZ); Pharmix s.r.o., 76701 Kromeriz (CZ)
(72) Erfinder: Kàña, Jan, 400011 Usti nad Labem (CZ); Marousek, Josef, 37006 Ceské Budëjovice (CZ); Rousar, Ivo, 10300 Praha 10 (CZ); Slaby, Frantisek, 53375 Dolni Redice (CZ)
(74) Vertreter: Markes, Libor

(57) **Zusammenfassung**

Anlage zur Verarbeitung von Phytomasse mittels kontinuierlicher Druckhydrolyse und darauffolgender Expansion des Hydrolysats ist durch eine Hochdruckpumpe (1) für zerschmettertes Material gebildet, die in einen, für Drücke bis 3,5 MPa dimensionierten Hochdruckhydrolyser (2) mündet, der einen geschlossenen Raum bildet, an dessen Eintritt sich ein Erwärmer (3) für das Material befindet, und der mit einer inneren Schnecke (4) für regulierten Materialtransport versehen ist, wobei sich an den Hochdruckhydrolyser (2) ein Expansionsdrehkreuz (5) anschließt, das in ein Expansionsgefäß (6) mit einem Brüdenabzug (7) mündet.

## Beschreibung

### Technisches Gebiet

Die Erfindung betrifft eine Anlage zur Verarbeitung von einen signifikanten Anteil an Phytomasse enthaltenden Materialien mittels kontinuierlicher Druckhydrolyse und darauffolgender Expansion des Hydrolysats.

### Bisheriger Stand der Technik

Die Pflanzen speichern Energie in Form von energieintensiven Bindungen, die bei der Synthese von Biopolymeren von hoher Molekülmasse entstehen. Es handelt sich namentlich um Zellulose, Hemizellulose und Stärke. Im Bereich der Biotechnologien und in der chemischen Industrie wird angestrebt, die in den Glukoseeinheiten der erwähnten Polymeren gespeicherte Energie freizusetzen, u. z. bei akzeptablem Kostenaufwand und minimaler negativer Auswirkung auf die Umwelt. Die Hydrolyse, als Verfahren der Verarbeitung von organischen Stoffen, entwickelt sich bereits seit Ende des 19. Jahrhunderts.

Aus Patentschriften sind mehrere Hydrolysenverfahren und dazu dienende Anlagen bekannt. Eine Anlage zur Produktion von Glukose, Äthanol, Furfural, Furan, Lignin, Essigsäure und Ameisensäure aus erneuerbaren Rohstoffen ist z.B. in der CZ 300865 beschrieben. Diese Anlage besteht aus einer Eintrittseinheit und einem Vorratsbehälter, der an eine Hydrolyseneinheit angeschlossen ist, die durch eine Beschickungspresse und ein System von Hydrolysern gebildet ist, das über einen Regelschieber und einen Satz von Produktbehältern mit einer Separationspresse verbunden ist. Dabei ist eine Überführungspresse zwischen der ersten und der zweiten Stufe der Hydrolyseneinheit eingeschaltet, die ein ständiges Druckgefälle zwischen den Stufen sicherstellt.

Die beschriebene Anlage, sowie die anderen bekannten Anlagen dieser Art haben zum Nachteil, dass es sich um Einzweckanlagen handelt. Deren Bauweise und Verfahren sind fast immer auf eine konkrete Phytomasse und auf eine Technologie orientiert. Manche Lösungen sind sehr kompliziert, weil sie sich darauf konzentrieren, fast alle Rohstoffe auszunutzen, die im Verfahren auftreten. Das resultiert in einer geringen Zahl der betriebenen Anlagen dieser Art als Folge von deren Energie- und Kostenintensität.

Der Erfindung liegt die Aufgabe zu Grunde, eine relativ einfache multifunktionale Anlage zu entwerfen, die im Stande ist, Phytomasse enthaltendes Material mittels eines, große Variabilität der Parameter zulassenden Verfahrens zu verarbeiten, ob mittels Hydrolyse oder Extrusion.

### Darstellung der Erfindung

Die obige Aufgabe wird durch eine Anlage zur Verarbeitung von einen signifikanten Anteil an Phytomasse enthaltenden Materialien mittels kontinuierlicher Druckhydrolyse und darauffolgender Expansion des Hydrolysats gelöst. Die Anlage ist durch eine Hochdruckpumpe für zerschmettertes Material gebildet, die in einen für Drücke bis 3,5 MPa dimensionierten Hochdruckhydrolyser mündet. Der Hydrolyser bildet einen geschlossenen Raum, an dessen Eintritt sich ein Erwärmer des Materials befindet und der mit einer inneren Schnecke für regulierten Materialtransport versehen ist. Dabei schließt sich an den Hydrolyser ein Expansionsdrehkreuz an, das in ein Expansionsgefäß mit einem Brüdenabzug mündet.

Zur Ausnutzung der in den Brüden enthaltenen Wärme kann die Anlage am Eintritt mit einem Wärmeaustauscher für die Materialerwärmung versehen werden, der an den Brüdenabzug angeschlossen ist, oder der Brüdenabzug kann direkt an den Erwärmer angeschlossen sein.

### Kurze Beschreibung der Abbildungen

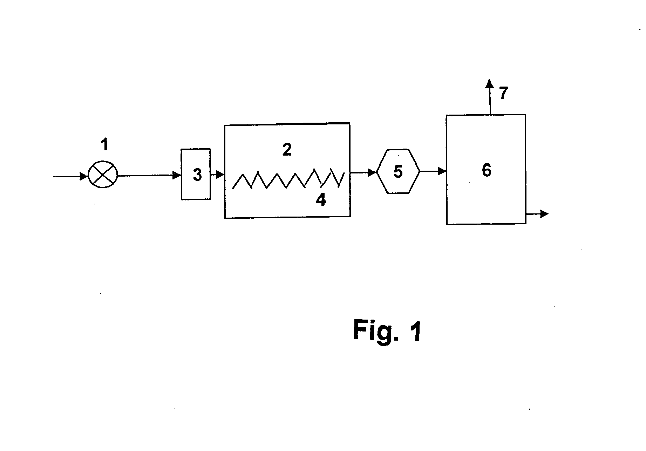

Die Erfindung wird nachstehend anhand von einem Ausführungsbeispiel näher erläutert. In der Zeichnung zeigt Fig. 1 einen Blockschaltbild einer erfindungsgemäßen Anlage zur Verarbeitung von einen signifikanten Anteil an Phytomasse enthaltendem Material.

### Ausführungsbeispiele der Erfindung

Die Anlage nach Fig. 1 zur kontinuierlichen Verarbeitung von einen signifikanten Anteil an Phytomasse enthaltenden Materialien mittels kontinuierlicher Druckhydrolyse und darauffolgender Expansion des Hydrolysats ist durch eine Hochdruckpumpe 1 für zerschmettertes Material gebildet, die in einen, für einen Druck von 3,5 MPa dimensionierten Hochdruckhydrolyser 2 mündet. Der Hochdruckhydrolyser **2** bildet einen geschlossenen Raum, an dessen Eintritt sich ein Erwärmer **3** für das Material befindet, und der mit einer inneren Schnecke **4** für regulierten Materialtransport versehen ist. An den Hochdruckhydrolyser **2** schließt sich dabei ein Expansionsdrehkreuz **5** an, das in ein Expansionsgefäß **6** mit einem Brüdenabzug **7** mündet.

Zur Ausnutzung der in den Brüden enthaltenen Wärme ist eine andere Ausführung der Anlage mit einem Wärmeaustauscher versehen zur Erwärmung des eintretenden Materials am Eintritt durch Brüdendämpfe. In einer weiteren Ausführung kann der Brüdenabzug **7** direkt an den Erwärmer **3** angeschlossen sein.

Die drei folgenden Beispiele zeigen die Eigenschaften und Vorteile des in der Anlage mittels Extrusion oder Hydrolyse verarbeiteten Materials bei der nachstehenden Ausnutzung.

Stroh, extrudiert bei 180°C und 1,5 MPa, wurde nachstehend zur Produktion des Biogases benutzt. Die Reaktionszeit im Reaktor reduzierte sich um 5 Tage im Vergleich zu nicht verarbeitetem Stroh.

Stroh, extrudiert bei 175°C und 1,4 MPa, wurde nachstehend einer Hydrolyse mit β-Glukosidase unterzogen. Nach der Hydrolyse lieferte das Produkt um 16 % mehr Glukose-Äquivalente als nicht verarbeitetes Stroh.

Stroh wies nach der Hydrolyse in der Anlage (bei 3% HNO₃ und 2,6 MPa) bei der Biogasproduktion eine um 10 Tage kürzere Reaktionszeit als eine Vergleichsprobe auf.

Die beschriebene multifunktionale Anlage lässt sich für eine breite Materialskala mit beliebigem Anteil an Phytomasse modifizieren, sowie für verschiedene Hydrolyse- und Extrusionsarten. Die Anlage ermöglicht biotechnologische Verarbeitung bei verschiedenen Verfahrensparametern. Das Wesentliche ist, dass die Expansion einstufig und sprungartig verläuft, und dass die Anlage einen geschlossenen Druckraum bildet.

## Patentansprüche

1. Anlage zur Verarbeitung von einen signifikanten Anteil an Phytomasse enthaltenden Materialien mittels kontinuierlicher Druckhydrolyse und darauffolgender Expansion des Hydrolysats, **dadurch gekennzeichnet, dass** sie durch eine Hochdruckpumpe (1) für zerschmettertes Material gebildet ist, die in einen, für Drücke bis 3,5 MPa dimensionierten Hochdruckhydrolyser (2) mündet, der einen geschlossenen Raum bildet, an dessen Eintritt sich ein Erwärmer (3) für das Material befindet, und der mit einer inneren Schnecke (4) für regulierten Materialtransport versehen ist, wobei sich an den Hochdruckhydrolyser (2) ein Expansionsdrehkreuz (5) anschließt, das in ein Expansionsgefäß (6) mit einem Brüdenabzug (7) mündet.

2. Anlage nach Anspruch 1, **dadurch gekennzeichnet, dass** sie am Eintritt mit einem Wärmeaustauscher für Materialerwärmung versehen ist, der an den Brüdenabzug (7) angeschlossen ist.

3. Anlage nach Anspruch 1, **dadurch gekennzeichnet, dass** der Brüdenabzug (7) direkt an den Erwärmer (3) angeschlossen ist.
